# EUROPEAN PATENT APPLICATION

(11) **EP 4 643 865 A1**
(43) Date of publication of application: **05.11.2025**
(21) Application number: 24755780.4
(22) Date of filing: 05.02.2024
(51) Int. Cl.: A61K 35/644, A61K 9/10, A61K 9/51, B82Y 5/00, B82Y 40/00

(54) **NANOSTRUCTURED AQUEOUS FORMULATION OF PROPOLIS EXTRACT AND THE USES THEREOF**

(30) Priority: 15.02.2023 BR 102023002791
(71) Applicant: Apis Vida Industria e Comerio de Produtos Farmaceuticos Ltda., 14707-016 Bebedouro (BR); Universidade de São Paulo - USP, 05508-220 São Paulo (BR)
(72) Inventor: MARCATO GASPARI, Priscyla Daniely, 14025-710 Ribeirão Preto (BR); KENUPP BASTOS, Jairo, 14030-000 Ribeirão Preto (BR); SILVA FERREIRA, Iasmin Rosanne, 14020-240 Ribeirão Preto (BR); MATIAZZI CORTEZ, Thais Fernanda, 14711-608 Bebedouro (BR); MATIAZZI, Luiz Henrique, 14700-335 Bebedouro (BR); PENA RIBEIRO, Victor, 14050-040 Ribeirão Preto (BR)
(74) Representative: Clarke, Modet y Cía., S.L.
(86) International application number: PCT/BR2024/050042
(87) International publication number: WO 2024/168409

(57) **Abstract**

The present invention relates to a nanostructured aqueous formulation containing a high content of propolis extract, in particular green or Brazilian red propolis extract, for use in pharmaceutical, food, cosmetic and veterinary formulations. The bioactives present in the propolis extract are responsible for its antioxidant, anti-inflammatory, antitumor and antiviral activities, which are enhanced and protected by the encapsulation of the extract in the lipid nanostructure.

## Description

### FIELD OF INVENTION

The present invention falls within the field of nanotechnology, more precisely in the pharmaceutical, food, cosmetic and veterinary areas, and refers to a nanostructured aqueous formulation of propolis extract with high solids content (Nano-AP). Said formulation can be used in liquid form or added to semi-solid or liquid bases to obtain a product with antioxidant, antimicrobial, anti-inflammatory, antitumor, antiviral activities for humans or in the veterinary area.

### BASIS OF THE INVENTION

Since ancient times, propolis has been used as a therapeutic agent, being known for its antioxidant, anti-inflammatory, antitumor, anti-allergic, antifungal and antimicrobial actions (Patel, 2015; Endo *et al.,* 2018; Daugsch *et al.,* 2008). Furthermore, it has been shown to be effective against cancers of the brain, head and neck, skin, breast, liver, pancreas, kidney, bladder, prostate, colon and blood (Patel, 2015).

Propolis is a resin formed by substances collected from various parts of plants by bees, mainly from the species *Apis mellifera,* mixed with their secretions, such as beeswax and saliva. It is used by bees to close openings in the hive and protect it from insects and microorganisms (Devequi-Nunes *et al.,* 2018; De Mendonça *et al.,* 2015; Reis *et al.,* 2019).

The composition of propolis varies depending on the geographic region and its plants, and is generally made up of 50% resins, rich in secondary metabolites, 30% waxes, 10% essential oils, 5% pollen and 5% other substances. More than 300 chemical compounds have already been identified, especially phenolic compounds and flavonoids (Endo *et al.,* 2018; De Mendonça *et al.,* 2015; Reis *et al.,* 2019; Devequi-Nunes *et al.,* 2018).

Propolis can vary depending on the flora of the geographic region where the bees collect the materials and the period in which it is collected, presenting a varied and complex chemical composition. Its geographical and plant origin is of great importance for quality control, standardization and therapeutic efficiency (Lustosa *et al.,* 2008), and is quite relevant in its chemical and biological composition (De Mendonça *et al*., 2015).

Propolis is known worldwide for its antioxidant, anti-inflammatory, antifungal, antiviral and antimicrobial properties (Sforcin *et al.,* 2011; Dantas *et al*., 2017).

Green propolis (PV) is the most widely used propolis worldwide. *Baccharis dracunculifolia* DC (Asteraceae), known as *"alecrim-do-campo"* or *"vassoura"* is the main botanical source of green propolis, so named because of its color (Búfalo *et al.,* 2010). As noted below, the main components of green propolis extract are phenolic compounds, especially those derived from cinnamic acid, such as artepelin C, baccharin and drupanin, in addition to other phenolic acids, such as caffeic and coumaric acid (Barretta *et al.,* 2017; Sousa *et al.,* 2007, Endo *et al.,* 2018).

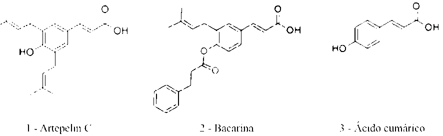

Brazilian red propolis (PVB) has also stood out in the world market for presenting a composition that is different from other types of propolis (Freires *et al.,* 2016). Its main botanical source is a species found in mangroves and on the coast of the Northeast region of the country, *Dalbergia ecasthophyllum* (Freires *et al.,* 2016; Reis *et al.,* 2019). The existence of polyprenylated derivatives of benzophenones in extracts of this propolis indicates the co-participation of another plant source, *Symphonia globulifera* (Aldana-Mejia *et al.,* 2021).

The compounds most related to its biological activity are secondary metabolites of the isoflavonoid type, such as Formononetin, Biochanin A and Vestitol, with benzophenones and other classes of compounds also found (Berretta *et al.,* 2017; Reis *et al.,* 2019).

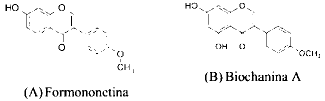

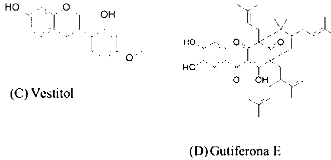

Despite the numerous advantages, the use of propolis extracts is limited due to its low bioavailability, low water solubility of its main components, strong odor characteristic of propolis and the presence of alcohol in the final formulation (Daudt *et al.,* 2013; Watkins *et al.,* 2015). Thus, the use of nanotechnology becomes an ally to overcome these limitations and enable the encapsulation of propolis extract and the obtaining of an aqueous dispersion with a high solids content and with properties superior to the pure extract. The encapsulation of molecules in nanostructures allows improving the performance and stability of active ingredients, increasing their bioavailability, solubilizing lipophilic compounds, providing a sustained release of the encapsulated active ingredient, directing it to the biological target, reducing toxicity, among other advantages (Mora-Huertas *et al.,* 2010; Daudt *et al.,* 2013).

Lipid nanostructures such as nanoemulsions and microemulsions stand out for their biocompatibility and for being biodegradable, as they present greater stability and a greater capacity to encapsulate lipophilic compounds in relation to, for example, liposomes, in addition to being easily prepared on a large scale (Miranda *et al.,* 2021, Singh *et al.,* 2017). Lipid nanostructures are versatile and can be incorporated into gels, creams, ointments, foams, etc. They can also be added to water to obtain flavored water, which can increase the stability of encapsulated natural compounds and prolong their release.

### STATE OF THE ART

Some prior art documents describe nanostructured formulations containing green or red propolis for applications in the dental and cosmetic fields, such as:

Document BR102012010441-5 A2 comprises a colloidal dispersion of glycolic propolis extract composed of a non-ionic surfactant and a viscosizing agent capable of reducing the hydrophobicity of the extract and its tendency to form precipitates in an aqueous medium, improving the miscibility of the structure without changing it. However, this document differs from the present invention, as it is composed of a polymeric micellar system, which is a nanostructured system different from the nanostructured system of the present invention, which is a microemulsion. The micellar system is composed of surfactant, propolis extract and water, while microemulsions are composed of surfactant, co-surfactant, oil, propolis extract and water. In addition, the aforementioned document used glycolic propolis extract, unlike the present invention, which used alcohol-free propolis extract.

Document CN103768103 B refers to an oil-water nanoemulsion comprising 1% propolis extract, solvent oil, surfactant and ultrapure water, wherein the solvent oil is selected from the group comprising mineral oil, vegetable oil, animal oil or synthetic oil. The surfactant is selected from the group comprising phospholipids, non-ionic surfactants, or a mixture thereof. In addition, the average particle size is 15 to 70 nm, preferably 15 to 50 nm, more preferably 15 to 20 nm. However, such document differs from the present invention by the type of nanostructure and method used. For example, in the aforementioned document, a nanoemulsion obtained by a high-energy method using a high-pressure homogenizer and high temperature (75 °C) was used, while in the present invention the microemulsion was obtained with low stirring speed and temperature (25-45°C) in order to preserve the compounds present in the propolis extract and contains 5 to 12%, preferably 8 to 12% of propolis extract.
The components of the nanostructure are also distinct, impacting differences in the physical-chemical and biological properties of the nanostructures.

Document BR102015016405-0 B1 describes polymeric nanoparticles containing red propolis extract and a nanopolymeric matrix composed of coating and dispersant/stabilizing polymers. The nanoencapsulating system is composed of a binary system with biocompatible, biodegradable properties that promote controlled release of the active ingredient, in addition to protecting dermocosmetic compositions from oxidative attack by the phenolic substances present in the propolis extract. Document BR102015033031-6 A2 describes, in addition to a process for obtaining it, a composition of nanoencapsulated products containing red propolis extract encapsulated in biopolymeric matrices that present antibacterial activity, which can be used to multiresistant infections control. This composition presents nanoencapsulated red propolis, composed of hydroethanolic extract of red propolis, aqueous phase and organic phase. It can be seen that both documents differ from the present invention due to the type of nanostructure used and its composition. In documents BR102015016405-0 and BR102015033031-6, polymeric nanoparticles were used using biodegradable polymers such as polycaprolactone (PCL) and poly(lactic acid-co-glycolic acid) (PLGA).

Furthermore, there are already nanostructured formulations containing pure green propolis for mouthwash, for example in document WO2013/163714, formulations of nanocapsules or polymeric nanospheres containing green propolis extract, as in document EP2633862A1, or red propolis extract, as in documents BR102016018124-0 and WO2018/023182 for applications in the pharmaceutical, cosmetic and agronomic areas, polymeric micelles containing green propolis extract to be used as a liquid dispersion, as in document CN1303899C, inclusion complex of propolis extract with cyclodextrin, as in documents WO2017/089842 and WO2012/073051. Furthermore, document CN101869234 describes the production of a nanoemulsion with green propolis extract prepared using organic solvents (ethanol and ethyl acetate) and the stabilizers Tween^{®}.

Document BR102018072871 refers to a polymer-coated microemulsion formulation containing green propolis extract or its isolates with a composition different from that of the present invention, with the aqueous phase composed of water, polyethylene glycol and bile salt, the oily phase composed of olive or sunflower oil and saturated fatty acids, and lecithin was used as a surfactant together with the bile salt. The microemulsion with the extract was obtained under high agitation, unlike the present invention in which the spontaneous formation process under low agitation was used in a single step. In addition, after the formation of the microemulsion, the inventors coated this nanostructure with chitosan or albumin, obtaining a nanostructured system different from the present invention. The change in the composition of the nanostructure can significantly alter the physical-chemical characteristics and, consequently, the biological effects of the nanostructure and its interaction with the organism (Ridolfo et al., 2021; Albanese et al., 2012).

It can be seen that the documents cited differ from the invention presented here in the type of nanostructure; chemical composition and physical-chemical characteristics, and the present invention would be the first to bring a nanostructured lipid system to encapsulate a high content of total solids of propolis extract, in particular, extract of green or Brazilian red propolis obtained in a single step, under low agitation and without the use of organic solvent.

Thus, the present invention brings as an innovation a nanostructured lipid system of microemulsion of easy scalability, which dispenses the use of organic solvent containing a high content of propolis extract (Nano-AP), in particular, extract of green or Brazilian red propolis, not limited to these extracts, for application as aqueous formulations in the pharmaceutical, food, cosmetic and veterinary areas with antioxidant, antimicrobial, anti-inflammatory, antitumor, antiviral activity including its action on the SARS-CoV-2 virus that causes COVID-19.

### SUMMARY OF THE INVENTION

The present invention aims to propose a nanostructured aqueous formulation containing a high content of propolis extract (Nano-AP), in particular, green propolis extract (Nano-APV) or Brazilian red propolis extract (Nano-APVB), aiming at its application in pharmaceutical, food, cosmetic and veterinary formulations. The bioactives present in the propolis extract are responsible for its antioxidant, antimicrobial, anti-inflammatory, antitumor and antiviral activities that are enhanced by the encapsulation of the extract in the microemulsion.

### BRIEF DESCRIPTION OF THE FIGURES

In order to obtain a total and complete visualization of the object of this invention, the figures to which references are made are presented, as follows.
Figure 1 graphically represents the analysis of the size distribution and polydispersity (PDI) of A) Nano-APV on day 30 (red curve) and after 400 days (green curve); and B) Nano-APBV on day 1 (red curve) and after 150 days (green curve), indicating the high stability of the aqueous formulations containing high extract content.
Figure 2 graphically represents the chromatogram obtained by HPLC analysis of the Nano-APV aqueous formulation.
Figure 3 graphically represents the chromatogram obtained by HPLC analysis of the commercial aqueous Extract 1.
Figure 4 graphically represents the chromatogram obtained by HPLC analysis of the commercial aqueous Extract 2.
Figure 5 graphically represents the antioxidant potential of the pure green propolis extract and Nano-APV measured by the DPPH radical reduction method, indicating the high antioxidant activity of Nano-APV.
Figure 6 graphically represents the cytotoxicity of pure green propolis extract and Nano-APV on human colorectal adenocarcinoma cells (Caco-2), indicating that Nano-APV has a cytotoxic effect on tumor cells that is superior to the pure extract.
Figure 7 graphically represents the evaluation of the antiviral effect of pure green propolis extract and Nano-APV on Caco-2 cells infected with the SARS-CoV-2 virus, indicating that Nano-APV has an effect on the SARS-CoV-2 virus that is superior to the pure extract.

### DETAILED DESCRIPTION OF THE INVENTION

The present invention refers to a nanostructured lipid formulation containing green or Brazilian red propolis extract with a high solids content that can be used as an aqueous dispersion or can be added to a base (semi-solid or liquid) and used in the pharmaceutical, cosmetic, food or veterinary areas. Said formulation comprises the following components:
- an oily phase consisting of 0.5 to 20%, preferably 2 to 10% of oil (lipid), 5 to 12%, preferably 8 to 12% of propolis extract, and - optionally, 8 to 18%, preferably 10 to 16% of lipophilic surfactants;
- an aqueous phase consisting of 12 to 35%, preferably 16 to 30% of hydrophilic surfactant, and water qsp with a pH between 6 and 9, preferably between 7.0 and 8.5;

The mixture of the aqueous phase with the oily phase occurs under agitation of 400-1500 rpm at a temperature of 25-50°C, preferably at 600-1000 rpm and 25-35°C.

The preparation of the formulation comprises the steps of:
a) Prepare the oily phase containing 0.5 to 20% oil and 5 to 12% propolis extract, which may or may not contain lipophilic surfactants (8 to 18%);
b) Prepare the aqueous phase containing 10 to 35% hydrophilic surfactant and water with a pH between 6 and 10;
c) Pour the aqueous phase into the oily phase under agitation of 400-1500 rpm at a temperature of 25 to 45°C;
d) Keep the system under agitation for 2 to 24 h.

The lipids are selected from the group consisting of soybean oil, capric/caprylic triglycerides, capryol^{®} 90, linseed oil, canola oil, grape seed oil, ben oil, wheat germ oil and coconut oil, preferably linseed oil, canola oil and soybean oil.

The surfactant is selected from the group consisting of polyoxyethylene-polyoxypropylene-polyoxyethylene copolymer (Pluronic^{®} F68 or 407), kolliphor^{®} HS15 or polysorbates (Tween^{®} 80, Tween^{®} 60, Tween^{®} 20) or Sorbitan monooleate (Span^{®} 60, Span^{®} 80, Span^{®} 20), preferably Kolliphor^{®} HS15, polysorbate 80 and sorbitan monooleate 80.

### Examples of the Invention

Variation in the proportions between the components of the lipid nanostructure as well as in the type of propolis extract lead to differences in the physical-chemical characteristics of the nanostructure obtained:

### Example 1

A nanostructured aqueous formulation containing the green propolis extract (Nano-APV) was prepared by mixing the aqueous phase composed of water with a pH between 6 and 9, preferably between 7 and 8, 16 to 30 % of surfactant (e.g. Kolliphor^{®} HS15), preferably 26%, with the oily phase composed of 2 to 12 % of linseed oil rich in Omega 3, 6 and 9, preferably 2.4% and with 5% to 11% of green propolis extract, preferably 11% under mechanical agitation (600-800 rpm) at 30-35 °C.

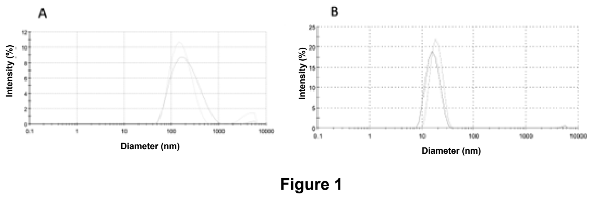

This formulation (Nano-APV) containing the green propolis extract with high solids content presented an average diameter between 120 and 189 nm; and polydispersity index (Pdl) of 0.224 to 0.353 (Figure 1A). The diameter of the Nano-APV nanostructures favors the skin permeation of active ingredients, making it interesting for the cosmetic area (Lemos et al., 2018; Pivetta et al., 2019). In addition, the dispersion was considered to have a low polydispersity index, being suitable for pharmaceutical, food, cosmetic and veterinary formulations. Its composition and reduced diameter favors the increase in the absorption of the extract compounds, making it interesting for oral products (Ali, Kolter, 2019). The formulation showed high physical stability (> 1 year), with the same diameter profile of the nanostructures being observed for 400 days (Figure 1A).

### Example 2

The potential of the nanostructured lipid system to encapsulate high content of different propolis extracts was evaluated, encapsulating the Brazilian red propolis extract (Nano-APVB).

Nano-APVB was prepared by mixing the aqueous phase composed of water with pH 7.5, 16-30% surfactant (e.g. Kolliphor^{®} HS15), preferably 20%, with the oily phase composed of 2 to 12 % of linseed oil rich in Omega 3, 6 and 9, preferably 3.5% and with 6% to 11% of Brazilian red propolis extract, preferably 6% under mechanical agitation (600-800 rpm) at 30-35 °C.

The nanostructured aqueous formulation containing Brazilian red propolis extract had a diameter between 15 and 50 nm, a polydispersity index (Pdl) of 0.05 to 0.157 and high stability (Figure 1B).

### Example 3

The Nano-APV aqueous dispersion from example 1 was used to obtain flavored water. The composition of the water consisted of 50-500mg/L of Nano-APV, preferably between 150-300 mg/L, with 0.04-0.10% sodium sorbate as a preservative. The pH of the water was adjusted between 5-8, preferably between 5-6.5, and then the flavored water was filtered through a 0.22 µm filter. The antioxidant activity, assessed by the DPPH radical reduction method, showed an EC₅₀ between 8 and 15 µg/mL.

### Example 4

The aqueous dispersion Nano-APV from example 1 or Nano-APVB from example 2 was used to obtain a topical formulation. To obtain the topical formulation, thickening and/or mucoadhesive agents were added to the Nano-APV or Nano-APVB dispersion. The composition of the topical formulation consisted of 98% Nano-APV and 2% quaternized guar gum or 2% Hydroxyethylcellulose, or a mixture of the thickening agents guar gum and hydroxypropylmethylcellulose (2:1) (2-9%). The formulation showed pseudoplastic rheological behavior with a decrease in viscosity with increasing shear rate (n < 1).

### Analysis of the phytochemical profile of the aqueous formulation of green propolis extract (Nano-APV)

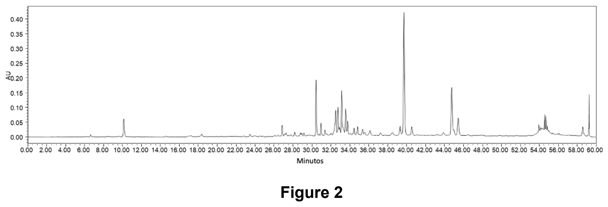

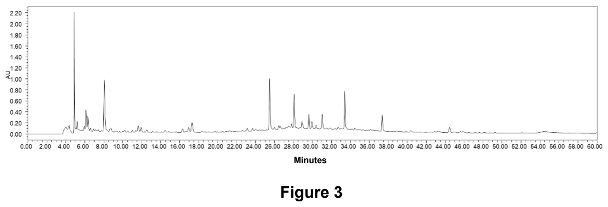

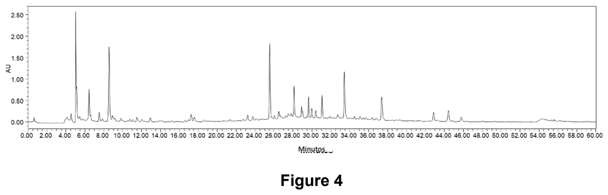

The phytochemical profile of the aqueous formulation of green propolis extract was evaluated by HPLC, and the main biomarkers were assessed (Figure 2). The phytochemical profile was compared to 2 commercial products of aqueous green propolis (Figures 3 and 4).

In the commercial aqueous extracts, it was not possible to identify the substances coumaric acid and artepelin C, which are compounds very relevant to the pharmacological activities of propolis. However, high concentrations of these compounds were quantified in the Nano-APV formulation (Table 1). Baccharin was found in all samples, but in the Nano-APV formulation a concentration 4 to 15 times higher than that quantified in the commercial aqueous products was observed. The same profile was observed for drupanin, dimethoxy-kaempferol (Table 1). Thus, the encapsulation of the green propolis extract in the lipid nanostructure preserves important propolis biomarkers, presenting a concentration of the same similar to the hydroalcoholic extract, which does not occur in commercial aqueous green propolis products. It is especially noteworthy that the new technology allows the production of commercial products in aqueous solution containing all the important propolis markers in the expected concentrations, thus ensuring their pharmacological activities, which were enhanced by being nanostructured.

**Table 1. Concentration of biomarkers in aqueous propolis sample**

| | **Concentration (µg/mL)** | | |
|---|---|---|---|
| | **Nano-APV** | **Commercial Aqueous Extract 1** | **Commercial Aqueous Extract 2** |
| Coumaric acid | 169.83 | ND | ND |
| Drupanin | 2499.74 | 334.79 | 735.04 |
| Dimethoxy-kaempferol | 470.91 | 287.54 | 253.55 |
| Artepelin C | 2278.23 | ND | ND |
| Baccharin | 443.28 | 26.57 | 95.56 |

| | | | |
|---|---|---|---|
| ND = Not detected | | | |

### Biological Activities

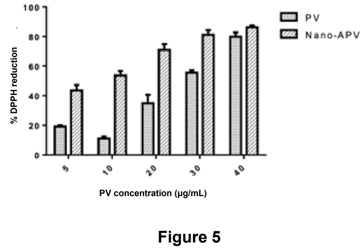

Nano-APV showed superior biological activity to pure green propolis extract, including antioxidant activity (Figure 5), being able to reduce 50% of the DPPH radical (EC₅₀) at a concentration of 8.9 µg/mL (3 times lower than that presented by pure propolis extract (EC₅₀ = 29.09 µg/mL). The oil, as well as the white nanostructure, did not present antioxidant activity, indicating that the formulation enhances the antioxidant activity of the propolis extract due to its reduced diameter and self-organization of the selected components of the nanostructure.

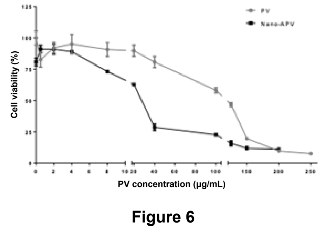

In addition, Nano-APV showed dose-dependent *in vitro* antitumor activity (Figure 6). Its cytotoxicity in human colorectal adenocarcinoma cells (Caco-2) was superior to pure propolis extract, presenting an IC₅₀ of 40.87 ± 0.034 µg/mL (2.5 times lower than that observed for pure green propolis extract, 101.9± 0.027 µg/mL). The white nanostructure showed low cytotoxicity with IC₅₀ greater than 150 µg/mL and the oil did not show toxicity in the concentration range evaluated. Thus, the nanostructured formulation shows a synergistic effect due to its composition and nanometric diameter that enhanced the antitumor activity of propolis. In addition, the Nano-APV formulation showed low toxicity in normal Vero cells (IC₅₀ = 184 µg/mL), indicating its selective effect on colorectal adenocarcinoma tumor cells.

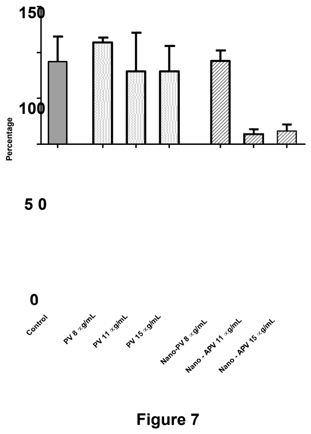

Nano-APV also showed dose-dependent *in vitro* antiviral activity, reducing the viral load of the SARS-CoV-2 virus by 87%, indicating its potential in the treatment of COVID-19. The antiviral activity of Nano-APV was superior to that of pure green propolis extract, which reduced only 21% of the viral load (Figure 7). The white nanostructure did not show antiviral activity, demonstrating that the Nano-APV formulation enhances the antiviral effect of propolis due to the self-organization of the selected components of the nanostructure associated with their reduced diameter.

Thus, the aqueous formulation, Nano-APV with a high content of total solids, showed high stability, preserved important biomarkers of propolis and enhanced the biological/pharmacological effects of the propolis extract, including antioxidant, antitumor and antiviral activity *in vitro.* In addition, the nanostructured lipid system was able to encapsulate a high content of Brazilian red propolis extract, indicating its versatility in encapsulating propolis extracts. The present invention, therefore, brings as an innovation a nanostructured lipid formulation of microemulsion obtained without the use of organic solvent as an encapsulation system for high content of propolis extracts in an aqueous medium that can enhance the pharmacological effects of propolis and can be used as an aqueous dispersion or can be incorporated into semi-solid or liquid formulations for the cosmetic, pharmaceutical, food and/or veterinary areas.

Therefore, the Nano-AP formulation uses the resources of nanotechnology to enhance the biological effects of the bioactives of propolis extracts, in addition to presenting the property of increased penetration and absorption due to its composition and diameter, greater stability and being easily incorporated into other pharmaceutical, cosmetic or food forms because they are in the form of aqueous dispersion.

### BIBLIOGRAPHIC REFERENCES

Aldana-Mejia, J.A.; Ccana-Ccapatinta, G.V.; Squarisi, I.S.; Nascimento, S.; Tanimoto, M.H.; Ribeiro, V.P. ; Arruda, C.; Nicolella, H.; Esperandim, T.; Ribeiro, A.B.; de Freitas, K.S.; da Silva, L.H.D.; Ozelin, S.D.; Oliveira, L.T.S.; Melo, A.L.A.; Tavares, D.C.; Bastos, J.K. Nonclinical Toxicological Studies of Brazilian Red Propolis and Its Primary Botanical Source Dalbergia ecastaphyllum. Chemical Research in Toxicology, 34, 1024-1033, 2021.

Ali, S., Kolter, K. Kolliphor® HS 15 - An enabler for parenteral and oral formulations. American Pharmaceutical Review 22 (1), 2019.

Berretta AA, Arruda C, Miguel FG. Functional Properties of Brazilian Propolis: From Chemical Composition Until the Market. In: Superfood and Functional Food - An Overview of Their Processing and Utilization. InTech, Chapter 4, 56-98, 2017.

Búfalo, M. C. et al. In vitro cytotoxic activity of Baccharis dracunculifolia and propolis against HEp-2 cells. Natural Product Research, 24, 1710-1718, 2010.

Dantas Silva RP, Machado BAS, Barreto G de A, et al. Antioxidant, antimicrobial, antiparasitic, and cytotoxic properties of various Brazilian propolis extracts. PLoS One.12, e0172585, 2017.

Daudt RM, Emanuelli J, Külkamp-Guerreiro IC, Pohlmann AR, Guterres SS. A nanotecnologia como estratpegia para o desenvolvimento de cosméticos. Ciência e Cultura 65, 28-31, 2013.

Endo S, Hoshi M, Matsunaga T, Inoue T, Ichihara K, Ikari A. Autophagy inhibition enhances anticancer efficacy of artepillin C, a cinnamic acid derivative in Brazilian green propolis. Biochemical and Biophysical Research Communications 497, 437-443, 2018.

Freires, I. A.; de Alencar, S. M.; Rosalen, P. L. A Pharmacological Perspective on the Use of Brazilian Red Propolis and Its Isolated Compounds against Human Diseases. European Journal of Medicinal Chemistry 110, 267-279, 2016.

Lemos, C.N., Pereira, F., Dalmolin, L.F., Cubayachi, C., Ramos, D.N., Lopez, R.F.V. Nanoparticles influence in skin penetration of drugs, in: Nanostructures for the Engineering of Cells, Tissues and Organs, Elsevier, Chapter 6, 187-248, 2018.

Mora-Huertas CE, Fessi H, Elaissari A. Polymer- based nanocapsules for drug delivery. International Journal of Pharmaceuticals 385,113-142, 2010.

Nascimento, T.G. do, Arruda, R.E. dos S., Almeida, E.T. da C., Oliveira, J.M. dos S., Basílio-Júnior, I.D., Porto, I.C.C. de M., Sabino, A.R., Tonholo, J., Gray, A., Ebel, R.E., Clements, C., Zhang, T., Watson, D.G. Comprehensive multivariate correlations between climatic effect, metabolite-profile, antioxidant capacity and antibacterial activity of Brazilian red propolis metabolites during seasonal study. Scientific Reports 9, 18293, 2019.

Pivetta, T.P., Silva, L.B., Kawakami, C.M., Araújo, M.M., Del Lama, M.P.F.M., Naal, R.M.Z.G., Maria- Engler, S.S., Gaspar, L.R., Marcato, P.D. Topical formulation of quercetin encapsulated in natural lipid nanocarriers: Evaluation of biological properties and phototoxic effect. Journal of Drug Delivery Science and Technology 53, 101148, 2019.

Reis, J. H. O. et al. Evaluation of the antioxidant profile and cytotoxic activity of red propolis extracts from different regions of northeastern Brazil obtained by conventional and ultrasoundassisted extraction. PlosOne, 14, 1-27, 2019.

Sforcin JM, Bankova V. Propolis: Is there a potential for the development of new drugs? J Ethnopharmacol. 2011;133(2):253-260. doi:10.1016/j.jep.2010.10.032

Singh, L., Kruger H.G., Maguire G.E.M., Govender T.; Parboosing R. The role of nanotechnology in the treatment of viral infections, Therapeutic Advances in Infectious Disease 4, 105-131, 2017.

Sousa JPB, Furtado NAJC, Jorge R, Soares AEE, Bastos JK. Perfis Fisico-Quimico e Cromatográfico de Amostras de Própolis Produzidas Nas Microrregiões de Franca (SP) e Passos (MG), Revista brasileira de farmacognosia 17, 2007.

Watkins R, Wu L, Zhang C, Davis RM, Xu B. Natural product-based nanomedicine: Recent advances and issues. International Journal of Nanomedicine 10, 6055-6074, 2015.

Ridolfo R, Tavakoli S, Junnuthula V, Williams DS, Urtti A, van Hest JCM. Exploring the Impact of Morphology on the Properties of Biodegradable Nanoparticles and Their Diffusion in Complex Biological Medium. Biomacromolecules 22, 126-133, 2021.

Albanese A, Tang PS, Chan WCW. The Effect of Nanoparticle Size, Shape, and Surface Chemistry on Biological Systems. Annual Review of Biomedical Engineering 14, 1-16, 2012

## Claims

1. Nanostructured lipid formulation of propolis extract **characterized by** the fact that it comprises:
- an oily phase consisting of 0.5 to 20%, preferably 2 to 10% of oil (lipid), 5 to 12%, preferably 8 to 12% of propolis extract, and optionally, 8 to 18%, preferably 10 to 16% of lipophilic surfactants;
- an aqueous phase consisting of 12 to 35%, preferably 16 to 30% of hydrophilic surfactant, and water qsp with a pH between 6 and 9, preferably between 7.0 and 8.5;
in which the mixing of the aqueous phase with the oily phase occurs under agitation of 400-1500 rpm, preferably 600-1000 at a temperature of 25-50 °C, preferably 25-35 °C.

2. Formulation, according to claim 1, **wherein** the lipids are selected from the group consisting of soybean oil, capric/caprylic triglycerides, capryol^{®} 90, linseed oil, canola oil, grape seed oil, ben oil, wheat germ oil and coconut oil, preferably linseed oil, canola oil and soybean oil.

3. Formulation according to claim 1, **wherein** the surfactant is selected from the group consisting of polyoxyethylene-polyoxypropylene-polyoxyethylene copolymer, mainly Pluronic^{®} F68 or 407) kolliphor^{®} HS15 or polysorbates, mainly Tween^{®} 80, Tween^{®} 60, Tween^{®} 20, or Sorbitan monooleate, mainly Span^{®} 60, Span^{®} 80, Span^{®} 20, preferably Kolliphor^{®} HS15, polysorbate 80 and sorbitan monooleate 80.

4. Use of the formulation as defined in any one of claims 1 to 3, **wherein** it is in liquid form or is added to semi-solid or liquid bases to obtain a product with antioxidant, antimicrobial, anti-inflammatory, antitumor, and antiviral activities.

5. Use of the formulation, according to claim 4, **wherein** it is for applications in the areas of pharmacy, food, cosmetics, and veterinary medicine.

6. Use of the formulation, according to claim 4, **wherein** it is also for addition to water to obtain flavored water.
